# EUROPEAN PATENT APPLICATION

(11) **EP 2 502 629 A1**
(43) Date of publication of application: **26.09.2012**
(21) Application number: 10830083.1
(22) Date of filing: 20.04.2010
(51) Int. Cl.: A61K 36/48, A61P 1/16, A23L 1/30, A23L 2/52

(54) **LIVER FUNCTION ENHANCER COMPOSITION**

(30) Priority: 16.11.2009 KR 20090110299
(71) Applicant: Nam, Jong Hyun, Songpa-gu, Seoul 138-110 (KR)
(72) Inventor: Nam, Jong Hyun, Songpa-gu, Seoul 138-110 (KR)
(74) Representative: Isarpatent
(86) International application number: PCT/KR2010/002462
(87) International publication number: WO 2011/059150

(57) **Abstract**

The present invention relates to a composition for the improvement of hepatic functions. More particularly, the present invention relates to a composition including an extract from Albizia julibrissin Durazz as an active ingredient.

## Description

### Technical Field

The present invention relates to a composition for improving a liver function. More particularly, the present invention relates to a composition for improving a liver function, including an Albizia julibrissin Durazz extract as an active ingredient.

### Background Art

The liver, often called the body's chemical factory, is one of the most important organs because given hepatic dysfunction, people lose their lives in the worst case.

This organ has a number of functions in the body which can be divided into three categories, including first, the metabolism of the three nutrients such as carbohydrates, lipids and proteins, second, the synthesis and storage of serum proteins such as albumin and coagulating factors, bile acid, phospholipids, cholesterol and enzymes, and third, the detoxification of waste or harmful substances

Viruses, alcohol, diabetes, obesity, drugs and harmful substances are known as causes of hepatic dysfunction (Mt Sinai J Med 2000; 67(1): 84-94).

Glutamate-pyruvate transaminase (hereinafter referred to as "GPT"), glutamate-oxaloacetate transaminase (hereinafter referred to as "GOT") and γ-glutamyl transpeptidase(γ-GTP) are enzymes that are released from the liver into the blood when hepatic dysfunction occurs due to these factors, and thus can be used to indicate hepatic dysfunction (M. Hayashi, et al., Nippon Yakuri gaku Zasshi, 100, 391-399(1992)).

It is known that a substance capable of decreasing GPT, GOT and γ-GTP activities can improve liver function. Korean Publicized Patent No. 2004-0084475 discloses a hepatic function improving agent comprising a powder of a mixture of milk vetch root, Codonopsis pilosula, Poria cocus, Licorice root, Polydonati rhizome, Maximowiczia typical, Chinese matrimony vine, an ethanol extract of the powder, a water extract of the powder, or a mixture of the extracts as an active ingredient. An extract from honeyberry fruits is described as such an active ingredient in Korean Registered Patent No. 699782 while Korean Registered Patent No. 771524 discloses an extract from a mixture of an oriental raisin fruit, Artemisia capillaris Thunb., Kudzu-vine, Citrus unshiu Markovich peel, Atractylodes japonica KOIDZ. et. KITAGAWA, licorice root and red ginseng.

The present invention addresses the improvement of hepatic function by use of an Albizia julibrissin Durazz extract suppressant of GPT, GOT and γ-GTP activities.

### Disclosure

### Technical Problem

It is an object of the present invention to provide a composition for improving hepatic functions.

Other and concrete technical features will be apparent from the following description.

### Technical Solution

Leading to the present invention, intensive and thorough research into the improvement of hepatic functions, conducted by the present inventor, resulted in the finding that an 80% ethanol extract, an 80% methanol extract or an ethyl acetate extract of Albizia julibrissin Durazz suppressed blood GPT, GOT and γ-GTP levels in animal tests and GPT, GOT and γ-GTP in clinical tests.

Based on the results of the tests, the present invention provides a composition for improving hepatic functions.

In accordance with the present invention, the composition for improving hepatic functions includes an Albizia julibrissin Durazz extract as an active ingredient.

As used herein, the term "active ingredient" means a component that can exhibit desired activity, alone or in combination with a carrier, which is itself not active.

The term "Albizia julibrissin Durazz extract," as used herein, is intended to include not only an extract from any part of a Albizia julibrissin Durazz plant, such as the leaves, stems, flowers, roots or a combination thereof, by use of a solvent such as ethanol, methanol, acetone, ethyl acetate, saturated n-butanol, chloroform, methylene chloride, water or a combination thereof, irrespective of extraction methods, but also a fraction of the extract in such a solvent. So long as it includes the immersion of the extraction target, such as Albizia julibrissin Durazz leaves, stems, flowers, roots or combination thereof, in a solvent, any extraction method may be used in the present invention. Examples of the extraction method include cold precipitation, refluxing, warming, and ultrasonication. Preferably, the "Albizia julibrissin Durazz extract" is obtained by immersing the extraction target selected from the group consisting of Albizia julibrissin Durazz leaves, stems, flowers, roots and combinations thereof in a solvent selected from the group consisting of water, ethanol, methanol, ethyl acetate or a combination thereof to give an extract of a concentrated liquid phase or a solid phase from which the solvent is removed.

Any amount of the active ingredient such as a Albizia julibrissin Durazz extract and the like may be contained in the composition for improving hepatic functions in accordance with the present invention so long as it effectively works to improve hepatic functions. The effective amount of the active ingredient varies depending on the use, formula, and formulation purpose of the composition, and is generally on the order of 0.001 % by weight to 99.900 % by weight based on the total weight of the composition. By the term "effective amount" is meant the amount of the active ingredient sufficient to result in inducing an improvement in liver function. Determining the amount experimentally is within the ordinary ability of a person skilled in the art.

In addition to the active ingredient such as an Albizia julibrissin Durazz extract, a supplemental ingredient in the composition for improving hepatic functions in accordance with the present invention may include natural products or compounds known for the activation of hepatic functions.

Examples of such natural products or compounds include powders or extracts of milk vetch roots, Codonopsis pilosula, Poria cocus, licorice roots, Polygonatum Rhizome, Schizandra chinensis Baillon, Lycium chinense Miller, Morus alba L, honeyberry fruits, oriental raisin (leaves, stems, flowers, roots, peels, fruits), Artemisia capillari, Kudzu-vine, Citrus unshiu Markovich peels, Atractylodes japonica KOIDZ. Ex Kitam, Alnus japonica Steud (leaves, stems, flowers, roots, peels, fruits), Rehmannia roots, Sorbus commixta (leaves, stems, flowers, roots, peels, fruits), and vegetable worms. Herein, the extract is as defined for the Albizia julibrissin Durazz, with the exception of the extraction target. The extract may be obtained from individual targets or a mixture of the targets.

According to one embodiment, the composition for improving hepatic functions of the present invention may be a food composition for beverages, particularly functional beverages.

The food composition may contain an additive such as a sweetener, a flavoring agent, a physiologically active substance, a mineral, etc. in addition to the active ingredient.

A sweetener is used to give a sweet taste to the composition and may be natural or synthetic. Preferable is a natural sweetener. Examples of the natural sweetener include corn syrup, honey, sucrose, fructose, lactose, maltose and other sugars.

A flavoring agent is adopted to enhance the taste or flavor of the composition and may be natural or synthetic. Preferable is a natural flavoring agent. A flavoring agent, if natural, may have the function of nutritional supplementation in addition to enhancing the flavor. Examples of the natural flavoring agent include those obtained from apple, lemon, mandarin, grape, strawberry, peach, green tea leaves, Polygonatum odoratum, bamboo leaves, cinnamon, chrysanthemum leaves, and/or jasmine. Other natural flavoring agents include those from ginseng (red ginseng), bamboo shoots, aloe vera, and/or ginkgo nuts. The natural flavoring agent may be in the form of a liquid concentrate or a solid extract. A synthetic flavoring agent may be used, and is exemplified by esters, alcohols, aldehydes and terpenes.

As the physiological active substance, catechins, such as catechin, epicatechin, gallocatechin and epigallocatechin, and vitamins, such as retinols, ascorbic acid, tocopherol, calciferol, thiamine and rivoflavin may be used.

As for the mineral, examples thereof include calcium, magnesium, chrome, cobalt, copper, fluorides, germanium, iodine, iron, lithium, magnesium, manganese, molybdenum, phosphorus, calcium, selenium, silicone, sodium, sulfur, vanadium, and zinc.

Optionally, the food composition of the present invention may further includes a preservative, an emulsifier, an acidulant, and a thickener in addition to the additives such as a sweetener, etc.

These agents such as preservatives, emulsifiers, etc. are used in as minimal an amount as possible to achieve the purpose of their addition. Numerically, their amount ranges from approximately 0.0005 % by weight to 0.5 % by weight based on the total weight of the composition.

Examples of the preservative useful in the present invention include calcium sorbate, sodium sorbate, potassium sorbate, calcium benzoate, sodium benzoate, potassium benzoate, and EDTA (ethylenediaminetetracetic acid).

Acacia gum, carboxymethylcellulose, xanthan gum, and pectin are emulsifiers that can be used in the present invention.

Representative among the acidulants are citric acid, malic acid, fumaric acid, adipic acid, phosphoric acid, gluconic acid, tartaric acid, ascorbic acid and acetic acid. The acidulant may be added for the purpose of suppressing microbial proliferation as well as enhancing the taste.

The thickener useful in the present invention may be exemplified by a suspending agent, a flocculant, a gel forming agent, and a swelling agent.

In accordance with another embodiment, the composition of the present invention may be used as a pharmaceutical composition.

The pharmaceutical composition according to the present invention includes a pharmaceutically acceptable carrier in addition to the active ingredient and may be formulated into oral dosage forms (tablets, suspensions, granules, emulsions, capsules, syrup, etc.), parenteral dosage forms (sterile injections, aqueous or oily suspensions, etc.), and topical application forms (solutions, creams, ointments, gels, lotions, patches, etc.).

As used herein, the term "pharmaceutically acceptable" means that a material does not interfere with the effectiveness of the biological activity of the active ingredients and is low enough in toxicity to be used on the subject.

Examples of the pharmaceutically acceptable carrier include lactose, glucose, sucrose, starch (e.g., corn starch, potato starch, etc.), cellulose and its derivatives (e.g., sodium carboxymethyl cellulose, ethyl cellulose, etc.), malt, gelatin, talc, a solid lubricant (e.g., stearic acid, magnesium stearate, etc.), calcium sulfate, vegetable oil (e.g., peanut oil, cotton seed oil, sesame oil, olive oil), polyol (e.g., propylene glycol, glycerine), alginic acid, emulsifiers (e.g., TWEENS), wetting agents (sodium lauryl sulfate), colorants, flavoring agents, stabilizers, antioxidants, preservatives, water, saline, and phosphate buffered saline. These carriers may be used, individually or in combination, according to the formulation of the pharmaceutical composition.

A suitable excipient may be also employed in the pharmaceutical composition of the present invention. For example, an excipient suitable for formulating the pharmaceutical composition of the present invention into an aqueous suspension may be a suspending agent or dispersant such as sodium carboxymethyl cellulose, methyl cellulose, hydropropylmethylcellulose, sodium alginate, or polyvinylpyrrolidone. When the pharmaceutical composition is formulated into an injection, Ringer's solution, or isotonic sodium chloride may be used as an excipient.

The pharmaceutical composition of the present invention may be administered via an oral route or a parenteral route such as a topical route.

The daily dose pharmaceutically composition of the present invention may be administered at a daily dose of from 0.001 ~ 150 mg/kg of body weight and in a single dose or in multiple doses per day. The dose of the pharmaceutical composition of the present invention may vary depending on various factors including the route of administration, the patient's age, gender and weight, and the severity of illness and thus must be in no way understood to limit the scope of the present invention.

### Advantageous Effects

As described hitherto, the composition for improving hepatic functions, including an extract from Albizia julibrissin Durazz is provided. The composition may be used as a food composition for beverages or as a pharmaceutical composition for tablets, capsules, liquid formulations, etc.

### Mode for Invention

A better understanding of the present invention may be obtained through the following examples which are set forth to illustrate, but are not to be construed as limiting the present invention.

### WORKING EXAMPLES: Preparation of Extract from Albizia julibrissin Durazz

### WORKING EXAMPLE 1: Preparation of Albizia julibrissin Durazz Extract 1

Sliced Albizia julibrissin Durazz including leaves, stems, roots, barks and fruits was immersed in 80 % ethanol at 80°C for 12 hours, followed by removal of the plant debris. The solution was concentrated at a reduced pressure to afford an extract in a solid phase.

### WORKING EXAMPLE 2: Preparation of Albizia julibrissin Durazz Example 2

An Albizia julibrissin Durazz extract was prepared in the same manner as in WORKING Example 1, with the exception that 80% methanol was used, instead of 80% ethanol.

### WORKING EXAMPLE 3: Preparation of Albizia julibrissin Durazz Example 3

An Albizia julibrissin Durazz extract was prepared in the same manner as in Working Example 1, with the exception that ethyl acetate was used, instead of 80% ethanol.

### EXPERIMENTAL EXAMPLES: Assays for Activity of Improving Hepatic Function

### EXPERIMENTAL EXAMPLE 1: Animal Assay for Activity of Improving Hepatic Function

To examine in vivo whether the Albizia julibrissin Durazz extracts prepared in the Working Examples are able to improve hepatic functions, hepatotoxicity was introduced into test animals by feeding high-fat foods thereto.

In this context, SD male rats, three weeks old, were divided into groups of 12. A high-fat food was fed alone to a control and in combination with the extracts to test groups for 10 weeks (see Table 1, below).

**TABLE 1**

| Composition of Food Fed to Rats | |
|---|---|
| Group | Composition of Diet (wt %) |
| Control | Basic diet (92%) + lard (7%) + Cholesterol(1.0%) |
| Test Group 1 | Basic diet (90%) + lard (7%) + Cholesterol (1.0%) + Extract of Working Example 1 (2.0%) |
| Test Group 2 | Basic diet (90%) + lard (7%) + Cholesterol (1.0%) + Extract of Working Example 2 (2.0%) |
| Test Group 3 | Basic diet (90%) + lard (7%) + Cholesterol (1.0%) + Extract of Working Example 3 (2.0%) |
| *Basic diet = AIN-93G diet{crude fat content 7%, FeedLab Inc.} | |

The animals were bred in a breeding room maintained at a temperature of 18±2°C and a relative humidity of 60% under a light/dark cycle of 12L/12D, and were fed ad libitum with diet and water.

After the breeding was completed, the activity of GPT and GOT, indicative of liver damage, was examined in the rats. Blood samples were taken from the rats and centrifuged. The sera thus obtained were measured for GPT and GOT activities using GPT and GOT kits (Bayer, USA, brand name ADVIA 1650) and the results are summarized in Table 1, below.

**TABLE 2**

| GPT and GOT Activities | | |
|---|---|---|
| Group | GPT(U/L) | GOT(U/L) |
| Control | 69.41±14.49 | 131.0±25.21 |
| Test Group 1 (Administered with Exact of Working Example 1) | 60.07±7.83* | 95.06±12.32* |
| Test Group 2 (Administered with Exact of Working Example 2) | 59.54±4.92* | 92.52±13.83* |
| Test Group 3 (Administered with Exact of Working Example 3) | 61.83±5.04* | 109.5±10.27* |
| Value = Mean±SD, * p<0.05 | | |

Results of all experiments were evaluated using one way ANOVA and Duncan multiple test with the level of significance set at 0.05..

### EXPERIMENTAL EXAMPLE 2: Clinical Test for Activity of Improving Hepatic Function

A drink was prepared by dissolving 100 g of a mixture of 80 wt% of the extract of Working Example 1 and 20 wt% of honey in 1,200 mL of water and ingested once a day for 8 weeks by 15 patients suffering from hepatic dysfunction. Just before ingestion and after ingestion for four and eight weeks, blood samples were taken, and measured for GPT, GOT and γ-GPT activities. Only the extract of Working Example 1 was used.

The results are summarized in Table 3, below.

**TABLE 3**

| GPT, GOT and γ-GTP Activities | | | |
|---|---|---|---|
| Enzyme | Before Ingestion | After Ingestion for 4 Weeks | After Ingestion for 8 Weeks |
| GPT (U/L) | 22.36±3.59 | 18.36±5.83* | 16.50±3.70*** |
| GOT (U/L) | 21.57±7.55 | 17.29±6.56* | 15.71±6.16*** |
| γ-GTP(U/L) | 34.07±25.11 | 27.36±25.30*** | 28.21±20.90** |
| * p<0.05 Value = Mean±SD, *** p<0.001, ** p<0.01, * p<0.05 | | | |

Results were evaluated using paired T-test, with the level of significance set at 0.05, 0.01 and 0.001.

## Claims

1. A composition for improving hepatic functions, including an Albizia julibrissin Durazz extract.

2. The composition of claim 1, wherein the Albizia julibrissin Durazz extract is prepared by subjecting Albizia julibrissin Durazz leaves, stems, flowers, roots, barks, fruits or a combination thereof to extraction with a solvent selected from among ethanol, methanol, acetone, ethyl acetate, saturated normal-butanol, chloroform, methylene chloride, water and a combination thereof.

3. The composition of claim 1, wherein the Albizia julibrissin Durazz extract is prepared by subjecting Albizia julibrissin Durazz leaves, stems, flowers, roots, barks, fruits or a combination thereof to extraction with a solvent selected from among ethanol, methanol, acetone, ethyl acetate, water and a combination thereof.

4. The composition of claim 1, further including a powder or an extract of at least one plant selected from the group consisting of a milk vetch root, Codonopsis pilosula, Poria cocus, a licorice root, Polygonatum Rhizome, Schizandra chinensis Baillon, Lycium chinense Miller, a honeyberry fruit, oriental raisin (leaf, stem, flower, root, bark, fruit), Artemisia capillari, Kudzu-vine, a Citrus unshiu Markovich peel, Atractylodes japonica KOIDZ. Ex Kitam, Alnus japonica Steud (leaf, stem, flower, root, bark, fruit), a Rehmannia root, Sorbus commixta (leaf, stem, flower, root, bark, fruit), vegetable worms and Morus alba L.

5. The composition of any one of claims 1 to 4, wherein the composition is a food composition.

6. The composition of claim 5, wherein the food composition is a beverage.

7. The composition of any one of claims 1 to 4, wherein the composition is a pharmaceutical composition.
